# EUROPÄISCHE PATENTANMELDUNG

(11) **EP 1 926 034 A1**
(43) Veröffentlichungstag der Anmeldung: **28.05.2008**
(21) Anmeldenummer: 07405159.0
(22) Anmeldetag: 04.06.2007
(51) Int. Cl.: G06F 19/00

(54) **Verfahren und Zuordnung von Objekten zu Personen sowie System zur Durchführung des Verfahrens**

(30) Priorität: 27.11.2006 CH 19052006
(71) Anmelder: InfoMedis AG, 6055 Alpnach (CH)
(72) Erfinder: Mezger, Tobias, 8214 Gächlingen (CH); Kern, Christian, 8048 Zürich (CH); Zemp, Rolf, 6055 Alpnach (CH)
(74) Vertreter: Nückel, Thomas

(57) **Zusammenfassung**

Bei dem erfindungsgemäßen Verfahren zur Zuordnung von Objekten zu Personen wird ein erstes Objekt (3), das einer bestimmten Person zugeordnet ist, mit einem Identifikationscode (PID) versehen. Für die Zuordnung eines zweiten Objekts (5; 6; 7; 10) zu der Person wird der Identifikationscode (PID) vom ersten Objekt (3) ausgelesen und auf das zweite Objekt (5; 6; 7; 10) kopiert.

## Beschreibung

### Technisches Gebiet

Die Erfindung betrifft ein Verfahren zur Zuordnung von Objekten zu Personen sowie ein System zur Durchführung des Verfahrens.

In Spitälern ist es regelmäßig erforderlich Patienten mit Blutbeuteln, Infusionen und Medikamenten zu versorgen. Diese Tätigkeiten oder einfachen Handlungen werden vom Personal des Spitals häufig durchgeführt. Dabei darf es allerdings nicht zu Verwechslungen kommen.

Im Folgenden werden unter dem Ausdruck "einfache Handlungen" Handlungen vom Personal des Spitals verstanden, die keine besonders hohe Qualifikation verlangen. Dazu gehört beispielsweise die oben erwähnte Verabreichung von Medikamenten, die bereits in den Dispenser für den Patienten einsortiert sind. Auch das Auswechseln einer Infusionsflasche oder eines Blutbeutels wird zu diesen einfachen Handlungen gezählt. Unsicher sind diese einfachen Handlungen deshalb, weil die Gefahr einer Verwechslung nicht unerheblich ist. In der Studie von M. Stäubli und J. Suter "Die Komplikationenliste der Schweizerischen Gesellschaft für Innere Medizin", Schweizerische Ärztezeitung, 2004; 85: Nr. 21, 1109 - 1116 wurde festgestellt, dass die Anzahl der einfachen, aber durchaus häufigen unsicheren Handlungen im Spital maßgeblich dafür ist, wie oft später Komplikationen mit häufig schwerwiegenden Folgen für den Patienten auftreten.

### Stand der Technik

Soll einem Patienten beispielsweise ein Medikament verabreicht werden, wird bisher der Patient für den das Medikament bestimmt ist, vom Personal vor der Verabreichung vorwiegend visuell identifiziert, vor allem dann, wenn der Patient nicht ansprechbar ist. Die visuelle Identifikation durch das Personal ist allerdings als eine unsichere Handlung einzustufen, weil keine Gewähr besteht, dass das Personal den Patienten auch richtig identifiziert. Auch die Zuordnung von Blutkonserven und Medikamenten zum jeweiligen Patienten sind unsichere Handlungen. Um zu prüfen, ob eine Blutkonserve diejenige ist, die für den Patienten vorgesehen ist, identifiziert das Personal zuerst den Patienten und prüft dann visuell, ob die Angaben auf der Blutkonserve zu diesem Patienten passen. Hierbei kann es jedoch zu einer falschen Zuordnung kommen, was wiederum zu schweren Komplikationen führen kann.

### Darstellung der Erfindung

Eine Aufgabe der Erfindung ist es daher, ein Verfahren und ein System anzugeben bei dem die Gefahr, dass ein Objekt dem falschen Patienten zugeordnet wird, minimiert wird.

Die Aufgabe wird durch ein Verfahren zur Zuordnung von Objekten zu Personen mit den Merkmalen gemäß Patentanspruch 1 gelöst.

Bei dem erfindungsgemäßen Verfahren zur Zuordnung von Objekten zu Personen wird mittels einer elektronischen Einrichtung ein Identifikationscode generiert und ein erstes Objekt, das einer bestimmten Person zugeordnet ist, mit einem Identifikationscode versehen. Für die Zuordnung eines zweiten Objekts zu der Person wird der Identifikationscode des ersten Objekt mittels eines Schreib-Lesegeräts ausgelesen und auf das zweite Objekt kopiert.

Die Aufgabe wird zudem durch ein System zur Durchführung des Verfahrens mit den Merkmalen gemäß Patentanspruch 16 gelöst.

Das erfindungsgemäße System zur Durchführung des soeben beschriebenen Verfahrens umfasst ein Armband zur Aufnahme eines Identifikationscodes sowie ein Schreibgerät, um das Armband mit dem Identifikationscode zu versehen. Zudem sind ein oder mehrere Objekte vorgesehen, die zur Aufnahme eines Identifikationscodes geeignet sind. Schließlich umfasst das System ein tragbares Schreib-Lesegerät, um den Identifikationscode zu lesen und um den Identifikationscode auf die Objekte schreiben zu können.

Vorteilhafte Weiterbildungen der Erfindung ergeben sich aus den in den abhängigen Patentansprüchen angegebenen Merkmalen.

Bei einer Ausführungsform des erfindungsgemäßen Verfahrens wird als erstes Objekt ein Armband verwendet. Das Armband hat den Vorteil, dass es permanent mit der zu i-dentifizierenden Person verbunden ist und damit eine permanente und fehlerfreie Zuordnung zwischen dem Armband und der Person gewährleistet wird.

Bei einer weiteren Ausführungsform des erfindungsgemäßen Verfahrens wird zum Auslesen, Kopieren und/oder Prüfen ein tragbares Schreib-Lesegerät verwendet.

Bei einer Weiterbildung des erfindungsgemäßen Verfahrens wird für die Zuordnung eines weiteren Objekts zu der Person zuerst der Identifikationscode von einem derjenigen Objekte ausgelesen, das bereits den Identifikationscode aufweist und dann auf das weitere Objekt kopiert.

Nach einem weiteren Merkmal der Erfindung wird der Identifikationscode auf einem auf dem Objekt angeordneten RFID-Tag gespeichert. Die Verwendung eines RFID-Tags hat den Vorteil, dass der Identifikationscode schnell und berührungslos auf dem Objekt gespeichert werden kann und ebenso berührungslos und schnell auch wieder ausgelesen werden kann.

Alternativ dazu besteht auch die Möglichkeit, bei dem erfindungsgemäßen Verfahren den Identifikationscode als Bar-Code auszudrucken und auf dem Objekt zu befestigen.

Vorteilhafterweise wird auf dem RFID-Tag auch die Art des Objekts gespeichert. So kann beispielsweise auf dem RFID-Tag gespeichert werden, ob es sich bei dem Objekt um ein Behältnis für Blut, ein Blutprodukt oder eine Infusion, einen Dispenser, ein Implantat, eine Akte oder ein medizinisches Gerät handelt.

Darüber hinaus kann bei dem erfindungsgemäßen Verfahren geprüft werden, ob der Identifikationscode von einem Objekt auf ein weiteres Objekt kopiert werden darf. Falls dies nicht erlaubt ist, wird der Kopiervorgang nicht durchgeführt. Handelt es sich beispielsweise bei dem einem Objekt um eine Blutkonserve, so darf der auf der Blutkonserve gespeicherte Identifikationscode nicht auf Proberöhrchen zur Entnahme von Blut kopiert werden. Stattdessen kann im Regelwerk festgelegt sein, dass das Kopieren des Identifikationscodes nur vom Armband des Patienten zum Proberöhrchen erfolgen darf.

Zur Lösung der Aufgabe wird ferner vorgeschlagen, dass geprüft wird, ob ein Objekt bereits einen Identifikationscode aufweist. Falls dies der Fall ist, wird der Kopiervorgang nicht durchgeführt.

Vorteilhafterweise wird bei dem erfindungsgemäßen Verfahren abhängig von der Art des Objekts festgelegt, ob dessen Identifikationscode auf ein weiteres Objekt kopiert werden darf.

Zudem ist es von Vorteil, wenn der Identifikationscode in einer Datenbank gespeichert wird.

Darüber hinaus kann bei dem erfindungsgemäßen Verfahren die Zuordnung eines jeden weiteren Objekts zu der Person in der Datenbank gespeichert werden.

Des Weiteren ist es bei dem erfindungsgemäßen Verfahren möglich, jeden Zugriff auf den Identifikationscode in einer Datenbank zu speichern. Die in der Datenbank abgelegten Daten können später auch zu statistischen Zwecken ausgewertet werden. Zudem können die Daten verwendet werden, um die patientenbezogenen Kosten zu ermitteln. Des Weiteren können die auf der Datenbank gespeicherten Daten verwendet werden, um die Anzahl der potentiellen Fehlzuordnungen zu ermitteln.

Bei dem erfindungsgemäßen Verfahren kann das weitere Objekt ein Behältnis für Blut, ein Blutprodukt, eine Infusion, Gewebeprobe, Ausscheidung oder Haare, ein Dispenser, ein Medikament, ein Implantat, eine Akte oder ein Gerät sein.

Schließlich kann das erfindungsgemäße Verfahren in einem Krankenhaus, einem Labor oder einer Arztpraxis verwendet werden.

### Kurze Beschreibung der Zeichnungen

Im Folgenden wird die Erfindung mit mehreren Ausführungsbeispielen anhand von 3 Figuren weiter erläutert.

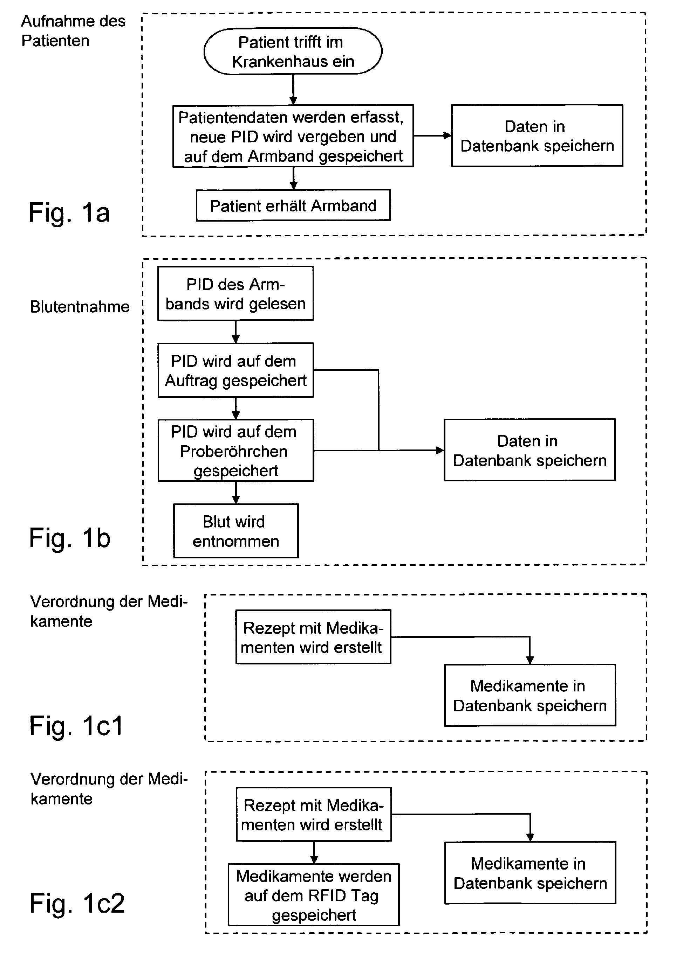

In den Figuren 1a bis 1i sind insgesamt 10 Teilprozesse in Form von Flussdiagrammen vereinfacht dargestellt. Es zeigen:
- Figur 1a: ein Flussdiagramm für die Aufnahme eines Patienten und die Vergabe eines persönlichen Identifikationscodes,
- Figur 1b: ein Flussdiagramm für den Teilprozess Blutentnahme,
- Figur 1c1: eine erste Ausführungsform für den Teilprozess Verordnung der Medikamente,
- Figur 1c2: eine zweite mögliche Ausführungsform für den Teilprozess Verordnung der Medikamente,
- Figur 1d: ein Flussdiagramm für den Teilprozess Zusammenstellung der Medikamente,
- Figur 1e: ein Flussdiagramm für den Teilprozess Untersuchung im Labor,
- Figur 1f: ein Flussdiagramm für den Teilprozess Lieferung aus dem Labor,
- Figur 1g: ein Flussdiagramm für den Teilprozess Behandlung 1 des Patienten,
- Figur 1h: ein Flussdiagramm für den Teilprozess Behandlung 2 des Patienten und
- Figur 1i: ein Flussdiagramm für den Teilprozess Entlassung des Patienten.
- Figur 2: zeigt anhand eines ersten Beispiels, wie die einzelnen Teilprozesse miteinander verknüpft sein können.
- Figur 3: zeigt anhand eines zweiten Beispiels, wie die einzelnen Teilprozesse miteinander verknüpft sein können.

### Wege zur Ausführung der Erfindung

Das Verfahren zur Zuordnung von Objekten zu Personen und das System zur Durchführung des Verfahrens dienen dazu beispielsweise Blutproben, Blutprodukte, Blutkonserven und Medikamente innerhalb einer beliebig langen Handlungskette im Spital und Labor oder einer Arztpraxis eindeutig einem Patienten zuzuordnen.

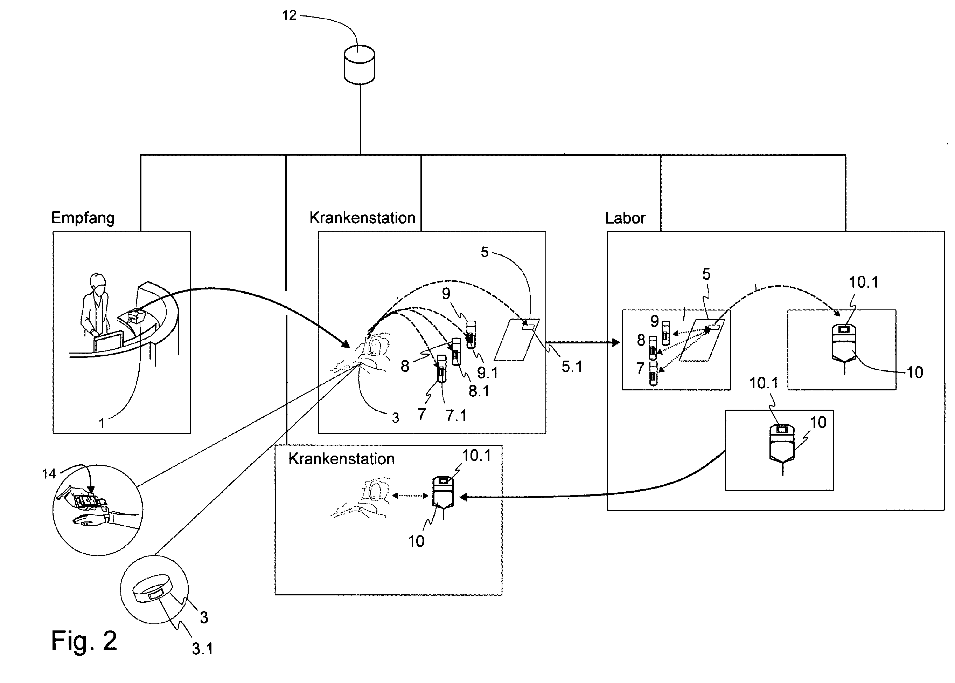

Im Folgenden werden einzelne Teilprozesse anhand der in den Figuren 1a bis 1i dargestellten Fußdiagramme beschrieben. In Figur 2 ist ein erstes Ausführungsbeispiel für eine typische Handlungskette dargestellt, die einige der in den Figuren 1a bis 1i gezeigten Teilprozesse beinhaltet.

In Figur 1a ist der Teilprozess "Aufnahme eines Patienten im Krankenhaus" dargestellt. Trifft der Patient im Krankenhaus ein, werden die Patientendaten am Empfang erfasst und ein neuer persönlicher Identifikationscode PID wird vergeben, der im Folgenden kurz als Identifikationscode bezeichnet wird. Anhand des Identifikationscodes PID ist der Patient nun eindeutig identifizierbar. Das bedeutet, dass für jeden Patienten ein neuer Identifikationscode PID vergeben wird. Der Identifikationscode PID ist eine Schlüsselinformation und kann eine Zahl, der Name des Patienten, eine Patienten- oder Fallnummer oder eine andere eindeutige Angabe über den Patienten enthalten. Der Identifikationscode PID wird auf einem Armband 3 gespeichert, das dem Patienten an das Handgelenk angelegt wird. Falls erforderlich, kann der Identifikationscode PID auf dem Armband 3 auch in verschlüsselter Form gespeichert sein. Dadurch wird ein Missbrauch des Identifikationscodes PID erschwert. Sowohl die Patientendaten als auch der Identifikationscode PID werden zusätzlich in einer Datenbank 12 gespeichert.

Um den Identifikationscode PID auf dem Armband 3 zu speichern, weist das Armband 3 einen Radio Frequency IDentification Tag 3.1, kurz RFID-Tag, oder einen Barcode auf. Der RFID-Tag 3.1 ist dabei Teil eines RFID-Systems. Das RFID-System umfasst den RFID-Tag 3.1, der auch als Transponder bezeichnet wird, der am Objekt angebracht ist und der es mit einem Identifikationscode PID kennzeichnet. Der RFID-Transponder 3.1 ist eine drahtlose Kommunikationseinheit, welche eingehende Funksignale aufnimmt und automatisch beantwortet. Der Begriff Transponder ist zusammengesetzt aus den Begriffen Transmitter und Responder. Das RFID-System umfasst zudem ein Lesegerät 14 zum Auslesen des Identifikationscodes des Transponders 3.1 und eine RFID-Middleware mit Schnittstellen zu weiteren EDV-Systemen und Datenbanken. Mit dem RFID-System lässt sich das Objekt automatisch identifizieren.

Im Unterschied zum Barcode kann der RFID-Transponder 3.1 jedoch zusätzliche Daten an verschiedenen Stationen aufnehmen und fungiert somit als variabler Datenträger am Objekt oder an der Person, der über eine Funkschnittstelle mit Radiowellen auslesbar ist. Typische Frequenzen für die Kommunikation sind < 134,2 kHz, 13,56 MHz und 868 beziehungsweise 915 MHz und 2,4 GHz. Passive RFID-Transponder arbeiten ohne Batterie und werden durch Induktion mit Energie versorgt. Aktive RFID-Transponder besitzen eine eigene Batterie, um ihr Signal, bestehend aus Kommunikationsdaten, Identifikationsdaten und Inhalt, zu einem Leseschreibgerät (Empfänger und Sender) zu senden.

Zusätzlich können mehrere RFID-Transponder gleichzeitig und gezielt vom Leseschreibgerät angesprochen werden (Antikollisionsfunktion). Vorteilhafter Weise durchdringen die Radiowellen, insbesondere bei tieferen Frequenzen, nichtmetallische Materialien. Die Schnittstellen und Beschreibungen der technischen Funktion von RFID-Transpondern sind der ISO 18000 zu entnehmen.

Die RFID-Transponder können auf Armbändern, Proberöhrchen, Laboraufträgen, Patientenakten oder Lieferzetteln Blutkonserven und Medikamentenbehältern oder Geräten befestigt sein.

Gemäß Figur 2 erfolgt als nächstes in der Handlungskette eine Blutabnahme beim Patienten. Der Teilprozess "Blutentnahme" ist in Figur 1b in Form eines Flussdiagramms gezeigt. Dabei wird in einem ersten Schritt der Identifikationscode PID vom Armband 3 des Patienten mit Hilfe eines tragbaren Schreib-Lesegerätes 14 gelesen und auf einem entsprechend vorbereiteten Auftragsformular 5 gespeichert. Das Auftragsformular 5 kann dazu mit einem RFID-Tag 5.1 ausgestattet sein. Anschließend wird der Identifikationscode PID des Patienten auf einem Proberöhrchen 7, das zur Blutentnahme bereitgehalten wird, gespeichert. Das Speichern des Identifikationscodes PID auf dem Proberöhrchen erfolgt ebenfalls vorzugsweise auf einem RFID-Tag 7.1, der auf dem Proberöhrchen 7 angebracht ist. Auf diese Weise wird sichergestellt, dass sowohl der Auftrag 5 als auch das Proberöhrchen 7 eindeutig dem Patienten zugeordnet sind. Anschließend kann das Personal dem Patienten Blut entnehmen und damit das Proberöhrchen 7 füllen. In der Datenbank 12 wird gespeichert, dass für den Patienten ein Auftragsformular 5 erstellt und ein Proberöhrchen 7 für das zu entnehmende Blut bereitgestellt wurde. Der Vorgang kann für weitere Proberöhrchen 8 und 9 wiederholt werden, die dafür ebenfalls mit RFID-Tags 8.1 beziehungsweise 9.1 ausgestattet sind.

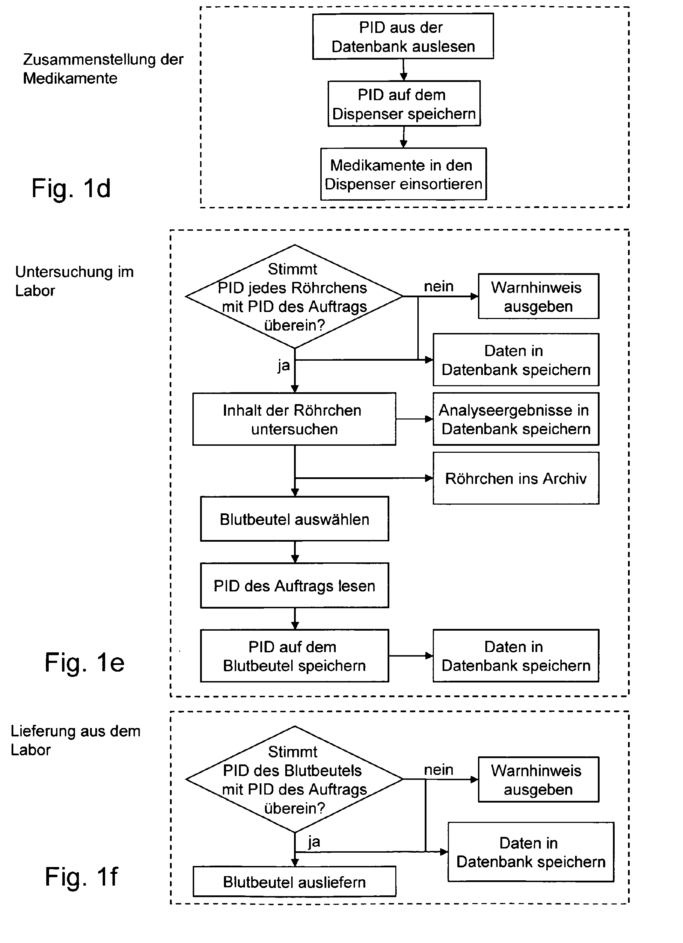

Im nächsten Schritt der Handlungskette wird, wie in Fig. 2 gezeigt, das entnommene Blut im Labor untersucht. Der entsprechende Teilprozess "Untersuchung im Labor" ist in Figur 1e als Flussdiagramm dargestellt. Zuerst wird im Labor überprüft, ob der Identifikationscode PID eines jeden Proberöhrchens 7, 8, 9 mit dem Identifikationscode PID des Auftrags 5 übereinstimmt. Ist dies nicht der Fall, wird ein Warnhinweis ausgegeben und dieser in der Datenbank 12 gespeichert. Stimmen hingegen der Identifikationscode PID eines jeden Röhrchens 7, 8, 9 und der Identifikationscode PID des Auftrags 5 überein, kann mit Sicherheit davon ausgegangen werden, dass alle Proberöhrchen 7, 8, 9 und der Auftrag 5 zum selben Patienten gehören. Das Ergebnis dieser Prüfung wird in der Datenbank 12 gespeichert. Anschließend werden die Proberöhrchen 7, 8, 9 untersucht und das Ergebnis der Untersuchung in der Datenbank 12 gespeichert und die Röhrchen 7, 8, 9 im Archiv aufbewahrt. Anhand des Analyseergebnisses wird dann der entsprechende Blutbeutel 10 ausgewählt, der Identifikationscode PID vom Auftrag 5 ausgelesen und auf dem Blutbeutel 10 gespeichert. Der Blutbeutel 10 kann dazu mit einem RFID-Tag 10.1 ausgestattet sein. Stattdessen kann der Identifikationscode PID auch als Bar-Code auf einem Etikett aufgedruckt und auf den Blutbeutel 10 aufgeklebt werden. Schließlich wird auch dieser Vorgang in der Datenbank 12 gespeichert.

Im nächsten Schritt der Handlungskette (in Fig. 2 nicht gezeigt) wird der Blutbeutel 10 zum Patienten gebracht. In Figur 1f ist dieser Teilprozess "Lieferung aus dem Labor" dargestellt. Wird beispielsweise im Operationssaal oder auf der Krankenstation Blut benötigt, wird ein entsprechender Auftrag an das Labor gesandt. Bevor der Blutbeutel 10 ausgeliefert wird, wird der Identifikationscode PID des Blutbeutels 10 mit dem Identifikationscode PID dieses Auftrags verglichen. Stimmen die beiden Identifikationscodes nicht überein, wird ein Warnhinweis ausgegeben. Anderenfalls wird die Übereinstimmung in der Datenbank 12 gespeichert und der Blutbeutel 10 an die entsprechenden Station ausgeliefert.

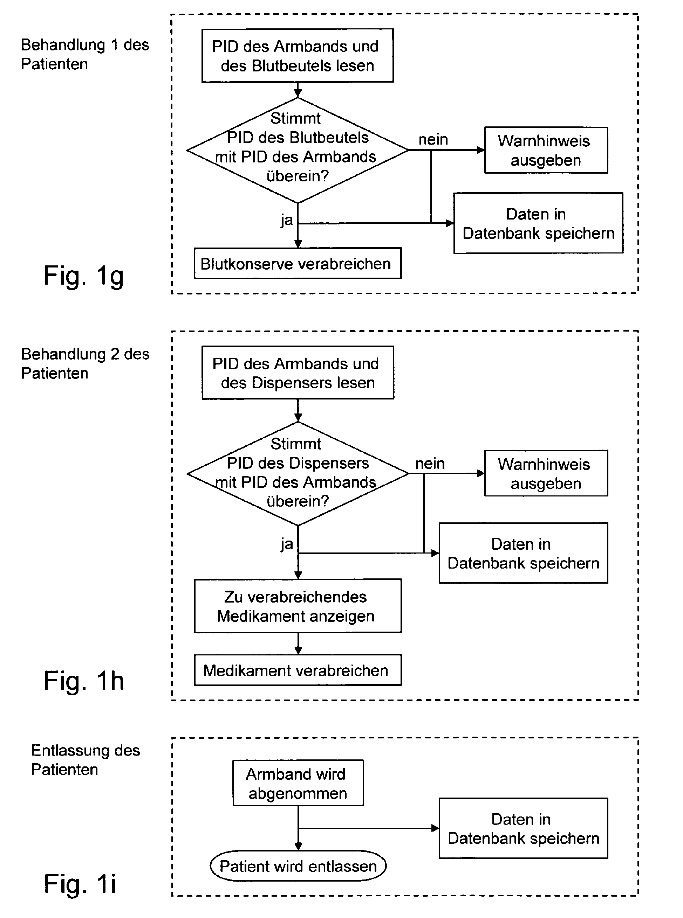

Im nächsten Schritt der Handlungskette wird, wie in Fig. 2 gezeigt ist, der Patient für die Transfusion mit Blut aus dem Blutbeutel 10 versorgt. In Figur 1g ist dieser Teilprozess als "Behandlung 1 des Patienten" bezeichnet und in Form eines Flussdiagramms dargestellt. Zuerst wird der Identifikationscode PID von Armband 3 des Patienten und vom Blutbeutel 10 ausgelesen und mit Hilfe des Schreib-Lesegerätes 14 miteinander verglichen. Stimmen die beiden Identifikationscodes nicht überein, wird vom Schreib-Lesegerätes 14 ein optischer und/oder akustischer Warnhinweis ausgegeben. Das Personal kann dann Maßnahmen ergreifen, um festzustellen, worin die Ursache für die unterschiedlichen Identifikationscodes liegt. Stimmen die beiden Identifikationscodes hingegen überein, wird die Transfusion freigegeben. Sowohl die Übereinstimmung als auch eine Nicht-Übereinstimmung werden in der Datenbank 12 gespeichert.

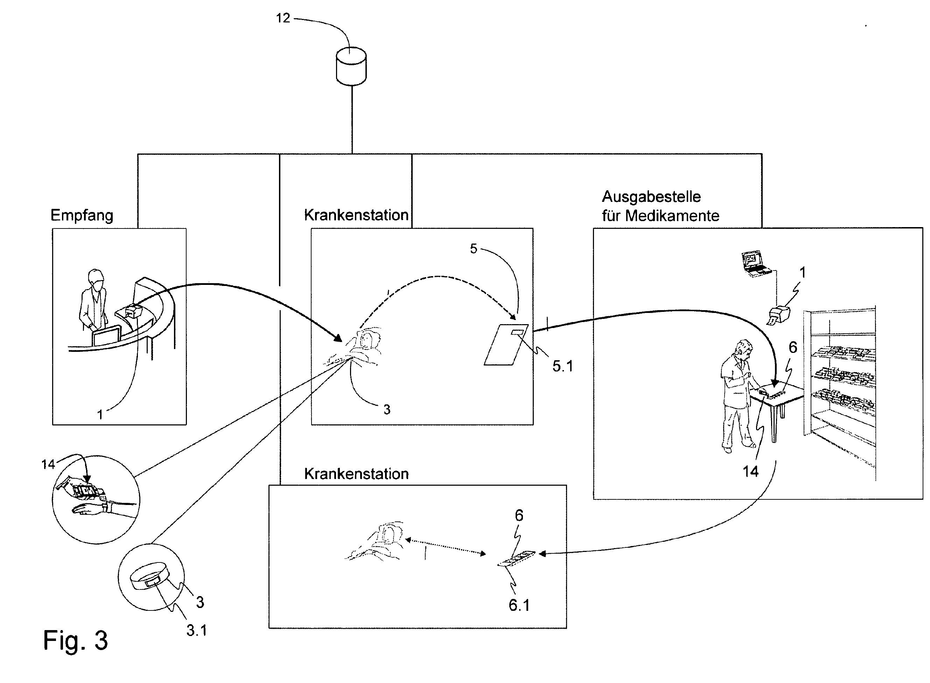

In Figur 3 ist ein zweites Ausführungsbeispiel für eine weitere typische Handlungskette dargestellt, die einige der in den Figuren 1a bis 1i gezeigten Teilprozesse beinhaltet.

Nachdem ein Patient am Empfang des Krankenhauses eingetroffen ist und seine Daten aufgenommen wurden, erfolgt sinngemäß wie im ersten Ausführungsbeispiel beschrieben, die Vergabe eines Identifikationscodes PID und die Speicherung dieses Identifikationscodes PID auf dem RFID-Tag des Armbands des Patienten. Dieser erste Schritt oder Teilprozess in der Handlungskette ist in Fig. 1a graphisch dargestellt.

Im nächsten Schritt der Handlungskette wird, wie im Teilprozess "Verordnung der Medikamente" gemäß Fig. 1c1 und 1c2 dargestellt, der Identifikationscode PID vom Armband des Patienten mit dem tragbaren Schreib-Lesegerät 14 ausgelesen und dieser Identifikationscode PID auf den RFID-Tag 5.1 des Auftragsformulars 5 kopiert. Dadurch ist das Auftragsformular 5 eindeutig dem Patienten zugeordnet. Der Arzt kann nun auf dem Auftragsformular 5 die für den Patienten vorgesehenen Medikamente notieren. Die auf dem Rezept oder Formular 5 notierten Medikamente werden dann beispielsweise vom Fachpersonal in der Datenbank 12 gespeichert.

Wie in Figur 1c2 gezeigt ist, können die Medikamente, die auf dem Rezept vom Arzt notiert wurden, zusätzlich auf dem RFID-Tag 3.1, der sich auf dem Armband 3 des Patienten befindet, gespeichert werden. Wenn dem Patienten später die Medikamente verabreicht werden sollen, kann geprüft werden, ob das zu verabreichende Medikament tatsächlich für diesen Patienten gedacht ist. Dazu werden mit dem tragbaren Schreib-Lesegerät 14 die auf dem RFID-Tag 3.1 gespeicherten Medikamente ausgelesen und mit dem zu verabreichenden Medikament verglichen. Falls das zu verabreichende Medikament in der Liste der zu verabreichenden Medikamente auftaucht, kann das Medikament verabreicht werden. Taucht das zu verabreichende Medikament allerdings nicht auf der Liste der zu verabreichenden Medikamente auf, ist das Personal gewarnt und kann der Ursache für diese Diskrepanz nachgehen.

Im nächsten Schritt der Handlungskette werden, wie in Fig. 3 gezeigt ist, in der Ausgabestelle für Medikamente die Medikamente für einen Patienten zusammen gestellt. In Figur 1d ist dieser Teilprozess als "Zusammenstellung der Medikamente" bezeichnet. Das Personal liest mit Hilfe des Schreib-Lesegeräts 14 den Identifikationscode PID aus der Datenbank aus. In einem nächsten Schritt wird mit Hilfe des Schreib-Lesegerätes 14 der Identifikationscode PID auf einem entsprechend vorbereiteten Dispenser 6 gespeichert. Der Dispenser 6 kann dafür beispielsweise einen programmierbaren RFID-Tag 6.1 tragen. Stattdessen kann der Dispenser 6 auch mit einer Etikette versehen sein, auf der der Identifikationscode PID als Bar-Code aufgedruckt ist. Nachdem der Dispenser 6 nun eindeutig dem Patenten zugeordnet ist, können die Medikamente in den Dispenser 6 einsortiert werden.

Im nächsten Schritt der Handlungskette werden, wie in Fig. 3 gezeigt ist, dem Patienten auf der Krankenstation die Medikamente verabreicht. In Figur 1h ist dieser Teilprozess als "Behandlung 2 des Patienten" bezeichnet und in Form eines weiteren Flussdiagramms dargestellt. Bevor dem Patienten ein Medikament verabreicht wird, werden der Identifikationscode PID des Armbands 3 und der Identifikationscode PID des Dispensers 6 mit dem tragbaren Schreib-Lesegerät 14 ausgelesen und die beiden Identifikationscodes werden miteinander verglichen. Stimmen sie nicht überein, wird ein Warnhinweis ausgegeben. Liegt hingegen eine Übereinstimmung vor, werden die Medikamenten freigegeben und die Freigabe in der Datenbank 12 gespeichert. Auf dem Anzeigefeld des Schreib-Lesegerätes 14 wird das zu verabreichende Medikament angezeigt. Das Personal kann dann das entsprechende Medikament entnehmen und dem Patienten verabreichen. Grundsätzlich können alle Aktionen sowohl bei Übereinstimmung als auch bei Nicht-Übereinstimmung in der Datenbank gespeichert werden.

Die einzelnen Schritte oder Glieder der Handlungskette werden durch die Übergabe von Schlüsselinformationen von einem RFID-Transponder zum nächsten RFID-Transponder geschlossen. Die Funktion ist unabhängig von einem zentralen Server beziehungsweise einer Datenbank, welche Entscheidungen im Sinne von "Blutkonserve/Medikament ist richtig zugeordnet" oder "Blutkonserve/Medikament ist falsch zugeordnet" treffen müsste.

Bei einer Ausführungsform des Verfahrens zur Übertragung von Schlüsselinformationen von Transponder zu Transponder werden programmierbare Transponder, das heißt Transponder mit variablen Datenspeichern verwendet.

Bei einem System, welches eine Information aus einem Transponder oder einem Barcode ausliest und diese an eine zentrale Datenbank liefert, wo sie auf ihre Zusammengehörigkeit hin überprüft wird, ist es erforderlich, dass die Datenbank jederzeit zur Verfügung steht. In einem solchen Systemen ist somit die Überprüfung einer Zuordnung vor Ort von der Verfügbarkeit der Datenbank abhängig. Wenn bei diesem zentralen System die Verbindung zur zentralen Datenbank, zum Beispiel über eine Wireless LAN-Verbindung nicht zur Verfügung steht, um Informationen an die Peripheriegeräte zu geben, kann gegebenenfalls bei einer Transfusion zu einem lebenskritischen Zeitpunkt des Patienten keine Überprüfung der Zuordnungen stattfinden. Daher ist es von Vorteil, wenn das Schreib-Lesegerät 14 zur Überprüfung der Zugehörigkeit eines Objekts zu einem Patienten unabhängig von der Netzwerk-Infrastruktur und der Datenbank arbeitet. Dadurch ist eine sehr hohe Sicherheit der zeitgerechten Zuordnung gegeben. Auch das Herunterladen von Patientendaten von der zentralen Datenbank auf ein Peripheriegerät ist nicht erforderlich. Alle Leseschreibgeräte 14 können offline betrieben werden. Die Leseschreibgeräte 14 können auch untereinander ausgetauscht werden. Die zentrale Datenbank kann beispielsweise ein Krankenhausinformationssystem (KIS) oder ein Laborinformationssystem (LIS) sein.

Die tragbaren Leseschreibgeräte 14 sind vorzugsweise mit einer Schnittstellensoftware für die Weitergabe von Daten an die zentrale Datenbank 12 oder zum Empfang von Daten von der zentralen Datenbank 12 ausgestattet.

Die mobile Leseschreibgeräte 14 sind als mobile Handcomputer, zum Beispiel als Personal Digital Assistant (PDA), Palm, Mobiltelefon, Laptop, Tablet PC ausgeführt. Die stationären Leseschreibgeräte 1 sind an einen stationären PC gekoppelt oder als vollständig integrierte Geräte ausgeführt. Hierzu zählen auch RFID-Drucker, welche das Armband 3 oder ein Etikett programmieren und mit einem Barcode oder visuell lesbaren Ziffern bedrucken. Die mobilen und stationären Leseschreibgeräte 1, 14 verfügen über eine RFID-Leseeinheit oder über eine Kombination aus RFID- und Barcode-Leseeinheit.

Das Leseschreibgerät 14 kann zum einen vergleichen oder prüfen, ob die Informationen, welche auf mehreren einander zugeordneten Transpondern gespeichert sind, identisch sind. Zum anderen kann das Leseschreibgerät 14 eine Information von einem bereits mit einem Identifikationscode PID versehenen Transponder, beispielsweise dem Transponder 3.1, auf einen weiteren Transponder, beispielsweise den Transponder 5.1, übertragen oder kopieren. So wird innerhalb einer beliebig langen Handlungs- oder Verfahrenskette eine Schlüsselinformation von einem Transponder zum nächsten weitergegeben. Die dabei genutzten Transponder werden in logischer Abfolge nacheinander programmiert. Dies wird durch eine entsprechende Vorprogrammierung der Transponder und entsprechende Berechtigungen der Leseschreibgeräte gewährleistet.

Das Armband 3 dient als Ausgangspunkt für alle weiteren Aktionen, zum Beispiel die Zuordnung von Blutkonserven, Blutprodukten, Medikamenten, der Abnahme von Blutproben und sonstigen Behandlungen zum Patienten. Geht das Armband 3 verloren, kann mit einer erneuten Identifikation des Patienten durch das Personal ein neues Armband mit dem ursprünglichen Identifikationscode PID programmiert werden.

Das Auftragsformular 5 kann entfallen, wenn auf visuell lesbare Informationen verzichtet wird. Im Labor wird die Zusammengehörigkeit der eingegangenen Proberöhrchen 7, 8, 9 und des Auftrags 5 geprüft. Die Proben werden anschliessend im Labor routinemässig bearbeitet. Aufgrund der Untersuchungsergebnisse werden eine oder mehrere passende Blutkonserven dem Patienten zugeordnet. Wenn eine oder mehrere Blutkonserven abgerufen werden und sie das Labor oder das Zwischenlager verlassen, kann ihre Zusammengehörigkeit untereinander mit der am Schreib-Lesegerät 14 vorgesehenen Funktion Prüfen überprüft werden. Die Prüfung kann zusätzlich Barcodes mit einbeziehen, sofern diese auf Formularen etc. aufgedruckt sind. Dies wäre beispielsweise bei der Auslieferung der Blutkonserven der Vergleich mit einem vorliegenden Auftrag zur Lieferung der richtigen Blutkonserve.

Der geschilderten Handlungsketten können variiert werden, indem zusätzliche Handlungsschritte dazwischen geschaltet werden. Beispielsweise kann als zusätzlicher Handlungsschritt die Liste im Operationssaal beziehungsweise die Krankenakte mit ihrem Barcode gegenüber dem RFID-Armband des Patienten auf ihre Zusammengehörigkeit hin überprüft werden. Bei der Zuordnung von Medikamenten wird durch den Arzt eine Verordnung auf einem Formular oder direkt elektronisch über die Eingabe in ein Terminal erfasst. Das Formular kann seinerseits wie bei der Blutabnahme einen RFID-Transponder enthalten, der direkt mit den Verschreibungsdaten programmiert wird. Das Formular 5 kann auch in Form einer Datei erzeugt werden. Das Formular 5 oder die Datei stehen bei der Medikamentenausgabe, das heißt bei der Befüllung der Medikamentenbehälter 6 zur Verfügung. Die Daten des Patienten werden bei der Zuteilung der Medikamente mit dem Leseschreibgerät 14 auf einen RFID-Transponder im oder am Dosierbehälter programmiert. Dieser Vorgang wird auch als Taufen bezeichnet. Wenn dieser Medikamentenbehälter 6 zum Patienten gebracht wird, wird der Identifikationscode PID im Dosierbehälter 6 mit dem Identifikationscode PID im Armband 3 des Patienten überprüft und damit die Zuordnung sichergestellt. Dieser Vorgang wird als Prüfen bezeichnet.

Die mobilen Leseschreibgeräte 14 und stationären Leseschreibgeräte 1 werden über einen integrierten oder separaten Computer beziehungsweise eine Rechnereinheit mit einem entsprechendem Programm gesteuert. Dieser Computer kann über WLAN oder LAN / serielle Schnittstelle an eine Datenbank 12 gekoppelt sein. Die Datenbank 12 empfängt von den Leseschreibgeräten 1 und 14 verfügbare Daten, beispielsweise über Aktionen an bestimmten Orten, Zeiten, Patienten bei Prüfungen und Taufen der Transponder. Auch eine Information über den Benutzer des Leseschreibgerätes 1, 14 kann weiter gegeben werden, insbesondere wenn dieser sich selbst durch eine geeignete Methode, zum Beispiel durch eine Smart Card, einen Barcode, PIN-Code, Fingerabdruck oder eine Spracherkennung am benutzten Leseschreibgerät 1, 14 identifiziert hat.

Die Datenbank 12 kann wiederum mit anderen Datenbanken, zum Beispiel einem Krankenhausinformationssystem oder einem Laborinformationssystem für den Datenaustausch verbunden sein. Alle von den Schreib-Lesegeräten 1 und 14 an die Datenbank 12 gelieferten Informationen dienen auch zur Dokumentation der einzelnen Handlungsschritte, wobei die Übertragung dieser Informationen an die Datenbank 12 automatisch erfolgt.

Die weitergegebene Schlüsselinformation auf den Transpondern kann verschlüsselt sein, wie dies zum Beispiel in Smart Cards bei der Zutrittskontrolle oder im Zahlungsverkehr eingesetzt wird. Dies verhindert eine mögliche missbräuchliche Veränderung der Daten. Die dabei verwendeten Protokolle der Luftschnittstelle sind in ISO 15693, ISO 14443, ISO 18000 festgelegt. Es können auch proprietäre, nicht standardisierte Protokolle verwendet werden.

Es können passive und aktive Transponder eingesetzt werden. Da die räumliche Reichweite des passiven Transponders relativ gering ist, kann der passive Transponder gezielt aktiviert werden, so dass dadurch Verwechslungen vermieden werden und die Belastung im Spital mit elektromagnetischen Störfeldern besonders gering gehalten wird. Zudem sind passive Transponder deutlich kostengünstiger und damit wirtschaftlicher einsetzbar als aktive Transponder. Aktive oder semiaktive Transponder können jedoch jederzeit in das System einbezogen werden.

Schließlich wird in Figur 1i der letzte Teilprozess "Entlassung des Patienten" grafisch dargestellt. Dabei wird dem Patienten das Armband abgenommen und der Vorgang in der Datenbank 12 gespeichert. Anschließend kann der Patient entlassen werden.

Die vorhergehende Beschreibung der Ausführungsbeispiele gemäß der vorliegenden Erfindung dient nur zu illustrativen Zwecken und nicht zum Zwecke der Beschränkung der Erfindung. Im Rahmen der Erfindung sind verschiedene Änderungen und Modifikationen möglich, ohne den Umfang der Erfindung sowie ihre Äquivalente zu verlassen.

### Bezugszeichenliste

- 1: Drucker oder stationäres Schreib-Lesegerät
- 3: Armband
- 3.1: RFID-Tag oder Barcode
- 5: Auftrag
- 5.1: RFID-Tag
- 6: Dispenser
- 7: Röhrchen
- 7.1: RFID-Tag
- 8: Röhrchen
- 8.1: RFID-Tag
- 9: Röhrchen
- 9.1: RFID-Tag
- 10: Blutbeutel
- 10.1: RFID-Tag
- 12: Speicher
- 13: Drucker
- 14: tragbares Schreib-Lesegerät

## Patentansprüche

1. Verfahren zur Zuordnung von Objekten zu Personen,
- bei dem mittels einer elektronischen Einrichtung (1) ein Identifikationscode (PID) erzeugt wird,
- bei dem ein erstes Objekt (3), das einer bestimmten Person zugeordnet ist, mittels der elektronischen Einrichtung (1) mit dem Identifikationscode (PID) versehen wird, und
- bei dem für die Zuordnung eines zweiten Objekts (5; 6; 7; 10) zu der Person der Identifikationscode (PID) des ersten Objekts (3) mittels eines Schreib-Lesegeräts (14) ausgelesen und auf das zweite Objekt (5; 6; 7; 10) kopiert wird.

2. Verfahren nach Patentanspruch 1,
bei dem als erstes Objekt (3) ein Armband verwendet wird.

3. Verfahren nach Patentanspruch 1 oder 2,
bei dem zum Auslesen, Kopieren und/oder Prüfen ein tragbares Schreib-Lesegerät (14) verwendet wird.

4. Verfahren nach einem der Patentansprüche 1 bis 3, bei dem für die Zuordnung eines weiteren Objekts (6; 7; 10) zu der Person zuerst der Identifikationscode (PID) von einem der Objekte (3; 5) ausgelesen wird, das bereits den Identifikationscode (PID) aufweist, und dann auf das weitere Objekt (6; 7; 10) kopiert wird.

5. Verfahren nach einem der Patentansprüche 1 bis 4,
bei dem der Identifikationscode (PID) auf einem auf dem Objekt (3; 5; 6; 7; 10) angeordneten RFID-Tag (3.1; 5.1; 6.1; 7.1; 10.1) gespeichert wird.

6. Verfahren nach einem der Patentansprüche 1 bis 4,
bei dem der Identifikationscode (PID) als Bar-Code ausgedruckt und auf dem Objekt (3; 6; 10) befestigt wird.

7. Verfahren nach Patentanspruch 5,
bei dem auf dem RFID-Tag (3.1;5.1) die Art des Objekts (3; 5; 6; 9; 10) gespeichert wird.

8. Verfahren nach einem der Patentansprüche 1 bis 7,
bei dem geprüft wird, ob der Identifikationscode (PID) von einem Objekt (3; 5; 6; 7; 10) auf ein weiteres Objekt (3; 5; 6; 7; 10) kopiert werden darf, und falls dies nicht erlaubt ist, der Kopiervorgang nicht durchgeführt wird.

9. Verfahren nach einem der Patentansprüche 1 bis 8,
bei dem geprüft wird, ob ein Objekt (3) bereits einen Identifikationscode (PID) aufweist, und falls dies der Fall ist, der Kopiervorgang nicht durchgeführt wird.

10. Verfahren nach einem der Patentansprüche 1 bis 8,
bei dem abhängig von der Art des Objekts (3; 5; 6; 10) festgelegt wird, ob dessen Identifikationscode (PID) auf ein weiteres Objekt (3; 5; 6; 10) kopiert werden darf.

11. Verfahren nach einem der Patentansprüche 1 bis 10, bei dem der Identifikationscode (PID) in einer Datenbank gespeichert wird.

12. Verfahren nach Patentanspruch 11,
bei dem die Zuordnung eines weiteren Objekts (5; 6; 7; 10) zu der Person in der Datenbank (12) gespeichert wird.

13. Verfahren nach Patentanspruch 11 oder 12,
bei dem ein Zugriff auf den Identifikationscode (PID) in einer Datenbank (12) gespeichert wird.

14. Verfahren nach einem der Patentansprüche 1 bis 13, bei dem das weitere Objekt (5; 6; 7; 10) ein Behältnis für Blut, ein Blutprodukt oder eine Infusion, ein Dispenser, ein Medikament, ein Implantat, ein Probengefäß, eine Akte oder ein Gerät ist.

15. Verwendung des Verfahrens nach einem der Patentansprüche 1 bis 14,
in einem Krankenhaus, einem Labor oder einer Arztpraxis.

16. System zur Durchführung des Verfahrens nach einem der Patentansprüche 1 bis 15,
- mit einem Armband (3) zur Aufnahme eines Identifikationscodes (PID),
- mit einem Schreibgerät (1), um das Armband (3) mit dem Identifikationscode (PID) zu versehen,
- mit einem oder mehreren Objekten (5; 6; 7; 10), die zur Aufnahme eines Identifikationscodes (PID) geeignet sind,
- mit einem tragbaren Schreib-Lesegerät (14), um den Identifikationscode (PID) zu lesen und um den Identifikationscode (PID) auf die Objekte (5; 6; 7; 10) schreiben zu können.
